(19) Europäisches Patentamt
European Patent Office
Office européen des brevets

(11) Veröffentlichungsnummer : **0 379 914 B1**

(12) **EUROPÄISCHE PATENTSCHRIFT**

(45) Veröffentlichungstag der Patentschrift : **08.09.93 Patentblatt 93/36**

(51) Int. Cl.[5] : **C08G 18/02,** C08G 18/79, C09D 175/04, C09D 179/04, C07D 251/34, C07D 273/04

(21) Anmeldenummer : **90100700.5**

(22) Anmeldetag : **13.01.90**

(54) **Verfahren zur Herstellung von modifizierten, Isocyanuratgruppen ausweisenden Polyisocyanaten und ihre Verwendung.**

(30) Priorität : **25.01.89 DE 3902078**

(43) Veröffentlichungstag der Anmeldung : **01.08.90 Patentblatt 90/31**

(45) Bekanntmachung des Hinweises auf die Patenterteilung : **08.09.93 Patentblatt 93/36**

(84) Benannte Vertragsstaaten : **AT BE CH DE ES FR GB IT LI NL SE**

(56) Entgegenhaltungen :
**EP-A- 0 043 651**
**EP-A- 0 166 173**
**EP-A- 0 235 388**
**EP-A- 0 295 926**

(73) Patentinhaber : **BAYER AG**
**D-51368 Leverkusen (DE)**

(72) Erfinder : **Scholl, Hans-Joachim, Dr.**
**Am Feldrain 5**
**D-5000 Köln 80 (DE)**

EP 0 379 914 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft ein neues Verfahren zur Herstellung von modifizierten, Isocyanuratgruppen aufweisenden Polyisocyanaten durch in Gegenwart von Kohlendioxid erfolgende katalytische Umsetzung eines Teils der Isocyanatgruppen von organischen Diisocyanaten mit aliphatisch und/oder cycloaliphatisch gebundenen Isocyanatgruppen und die Verwendung der Verfahrensprodukte, gegebenenfalls in blockierter Form, als Isocyanatkomponente in Polyurethanlacken.

Bei den erfindungsgemäßen Verfahrensprodukten handelt es sich im allgemeinen um Gemische, die neben Isocyanuratgruppen aufweisenden Polyisocyanaten der idealisierten Formel (II) Oxadiazintriongruppen aufweisende Diisocyanate der idealisierten Formel (I) und Iminooxadiazindiongruppen der Formel (III) aufweisen.

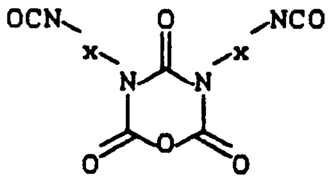

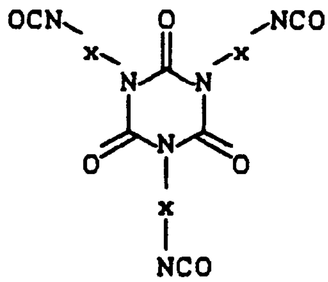

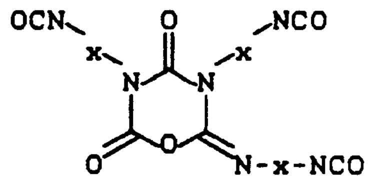

(I) (II) (III)

In diesen Formeln steht x jeweils für den Kohlenwasserstoffrest des zur Herstellung der modifizierten Polyisocyanate eingesetzten (cyclo)aliphatischen Diisocyanats. Neben den Tri- bzw. Diisocyanaten der genannten allgemeinen Formeln können in den Verfahrensprodukten auch noch höhere Homologe vorliegen, die pro Molekül mehr als einen heterocyclischen Ring der genannten Art aufweisen

Ein Verfahren zur Herstellung von derartigen Polyisocyanatgemischen ist neu. Zwar sind eine große Anzahl von Verfahren zur Trimerisierung organischer Isocyanate bekanntgeworden (J.H. Saunders und K.C. Frisch, Polyurethanes Chemistry and Technology, S. 94 ff (1962)), wobei als Katalysatoren insbesondere starke organische Basen eingesetzt werden. Bei Verwendung von organischen Diisocyanaten und Abbruch der Trimerisierungsreaktion, bevor alle Isocyanatgruppen des Ausgangsdiisocyanats abreagiert haben, entstehen bei dieser Umsetzung Isocyanuratgruppen aufweisende Polyisocyanate, die wertvolle Ausgangsmaterialien für den Polyurethanchemiker darstellen und insbesondere in Polyurethanlacken als Polyisocyanatkomponente zum Einsatz gelangen. Die Kombination dieser Trimerisierungsreaktion mit der in DE-AS 1 670 666 beschriebenen Herstellung von Oxadiazintriongruppen aufweisenden Polyisocyanaten, d.h. die Herstellung von Polyisocyanatgemischen der Art der erfindungsgemäßen Verfahrensprodukte ist in EP-A-0 043 651 beschrieben. Beim Verfahren dieser Vorveröffentlichung müssen jedoch unterschiedliche Katalysatoren für die Trimerisierungsreaktion einerseits und die unter Ringbildung ablaufende Kohlendioxidanlagerung andererseits eingesetzt werden. Für die letztgenannte Reaktion müssen hierbei physiologisch bedenkliche Phosphor- und Arsenverbindungen in beträchtlichen Mengen verwendet werden. Dies ist insofern nicht überraschend, als einerseits Kohlendioxid, ein Säureanhydrid, die basisch katalysierte Trimerisierung hindert und andererseits beim Verfahren gemäß DE-AS 1 670 666 Kohlendioxid permanent im Überschuß vorhanden sein muß, damit die Bildung von störenden, zu einem unerwünschten Viskositätsaufbau führenden Nebenprodukten vermieden wird.

Wie jetzt überraschend gefunden wurde, gelingt es mit dem nachstehend näher beschriebenen erfindungsgemäßen Verfahren beide an sich vorbekannten Modifizierungsreaktionen vorteilhaft unter Verwendung von physiologisch weit weniger bedenklichen Katalysatoren in beträchtlich reduzierten Konzentrationen zu kombinieren, so daß modifizierte, Isocyanuratgruppen aufweisende Polyisocyanate mit gezielt und variabel einstellbarer Zusammensetzung erhalten werden, die sich durch eine Reihe vorteilhafter Eigenschaften wie beispielsweise Farblosigkeit, gute Verträglichkeit mit den üblichen Polyhydroxylverbindungen und niedrige Viskosität auszeichnen. Beim erfindungsgemäßen Verfahren werden für beide Reaktionen als Katalysatoren quartäre Ammonium- oder Phosphoniumfluoride in vergleichsweise geringen Konzentrationen verwendet, wobei die Phosphoniumfluoride weit weniger physiologisch bedenklich sind als die in der letztgenannten Vorveröffentlichung empfohlenen Phosphine und zudem vorzugsweise völlig durch die entsprechenden Ammoniumfluoride ersetzt werden können.

In der EP-A-0 295 926 werden für eine völlig andersartige Reaktion, nämlich die Umsetzung von zyklischen Carbonaten mit Polyisocyanaten u.a. Gemische aus Kaliumfluorid und Benzyltriammoniumchlorid bzw. Tetrabutylphosphoniumchlorid empfohlen. Es handelt sich somit um andere Katalysatoren für eine andere Reaktion.

Gegenstand der Erfindung ist ein Verfahren zur Herstellung von modifizierten, Isocyanuratgruppen aufweisenden Polyisocyanaten mit aliphatisch und/oder cycloaliphatisch gebundenen Isocyanatgruppen durch chemische Modifizierung eines Teils der Isocyanatgruppen von organischen Diisocyanaten mit aliphatisch und/oder cycloaliphatisch gebundenen Isocyanatgruppen in Gegenwart eines, die Trimerisierung beschleunigenden Katalysators und Abbruch der Reaktion beim jeweils erwünschten Umsetzungsgrad, dadurch gekennzeichnet, daß man

(i) als Katalysator quartäre Ammonium- oder Phosphoniumfluoride verwendet und

(ii) die Modifizierungsreaktion in Gegenwart von zumindest zeitweise während der Umsetzung in das Reaktionsgemisch eingeleitetem Kohlendioxid durchführt.

Gegenstand der Erfindung ist auch die Verwendung der modifizierten, Isocyanuratgruppen aufweisenden Polyisocyanate gegebenenfalls in mit Blockierungsmitteln für Isocyanatgruppen blockierter Form als Isocyanatkomponente in Polyurethanlacken.

Für das erfindungsgemäße Verfahren von erfindungswesentlicher Bedeutung ist die Verwendung von quartären Ammonium- oder Phosphoniumfluoriden als Katalysatoren. Grundsätzlich geeignet sind alle beliebigen quartärneren Ammonium- oder Phosphoniumfluoride. Die Substituenten am Stickstoff- bzw. Phosphoratom können Alkyl-, Aryl- oder Aralkylgruppen oder eine beliebige Kombination derartiger Gruppen sein. Auch quartärnere Fluoride auf Basis von heterocyclischen Aminen sind geeignet. Zu den bevorzugten Katalysatoren gehören Verbindungen der allgemeinen Formel

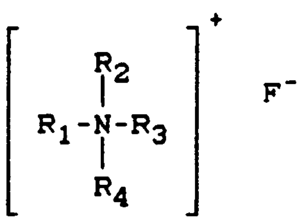

für welche

$R_1$, $R_2$, $R_3$ und $R_4$ für gleiche oder verschiedene Reste stehen und Alkylgruppen mit 1 bis 18, vorzugsweise 1 bis 10 Kohlenstoffatomen bedeuten, wobei einer der Reste auch für einen araliphatischen Rest mit 7 bis 15 Kohlenstoffatomen stehen kann, und wobei die Summe der Kohlenstoffatome der vier Reste vorzugsweise bei 10 bis 40 liegt, oder wobei die Reste $R_1$ und $R_2$ und gegebenenfalls gleichzeitig die Reste $R_3$ und $R_4$ jeweils zusammen mit dem Stickstoffatom einen gegebenenfalls weitere Heteroatome enthaltenden Ring mit vorzugsweise 6 Ringatomen bilden können.

Zu den bevorzugten Katalysatoren gehören insbesondere auch Verbindungen der zuletzt genannten allgemeinen Formel, für welche

$R_1$, $R_2$ und $R_3$ für gleiche oder verschiedene Alkylreste mit 1 bis 18, insbesondere 1 bis 4 Kohlenstoffatomen, besonders bevorzugt für Methylgruppen stehen und

$R_4$ für einen Benzylrest steht.

Zu den geeigneten bzw. bevorzugt geeigneten Katalysatoren gehören beispielsweise Tetramethylammoniumfluorid, Tetraethylammoniumfluorid, Tetra-n-propylammoniumfluorid, Tetra-n-butylammoniumfluorid, N-Methyl-N,N,N-trialkylammoniumfluorid mit $C_8$-$C_{10}$-Alkylresten, N,N,N-Trimethyl-N-cyclohexylammoniumfluorid, N,N,N-Trimethyl-N-benzylammoniumfluorid, N,N,N-Triethyl-N-benzylammoniumfluorid, N,N,N-Trimethyl-N-phenylammoniumfluorid, N,N,N-Trimethyl-N-stearylammoniumfluorid, N,N'-Dimethyl-triethylendiamin-difluorid oder N-Methyl-triethylendiamin-monofluorid. Beliebige Gemische der beispielhaft genannten Katalysatoren können selbstverständlich ebenfalls verwendet werden.

Besonders bevorzugt sind N-Methyl-N,N,N-trialkylammoniumfluorid mit $C_8$-$C_{10}$-Alkylresten, N,N,N,N-Tetra-n-butylammoniumfluorid und N,N,N'-Trimethyl-N-benzylammoniumfluorid.

Zu den geeigneten Phosphoniumfluoriden, die im Vergleich zu den Ammoniumfluoriden im allgemeinen weniger bevorzugt sind, gehören die entsprechenden Phosphoniumverbindungen, d.h. Verbindungen der

obengenannten allgemeinen Formel, für welche das zentrale Stickstoffatom durch ein Phosphoratom ersetzt ist und die Reste $R_1$ bis $R_4$ die genannte Bedeutung haben. Beispiele sind Tetramethylphosphoniumfluorid, Tetraethylphosphoniumfluorid oder Tetra-n-butylphosphoniumfluorid.

Die zum Einsatz gelangenden Katalysatoren können beispielsweise durch Umsetzung von

a) Alkalifluoriden, vorzugsweise Natrium- oder Kaliumfluorid, besonders bevorzugt Kaliumfluorid mit

b) quartären Ammonium- oder Phosphoniumsalzen von beliebigen Säuren mit Ausnahme der Fluorwasserstoffsäure, vorzugsweise starke Mineralsäuren, besonders bevorzugt Chlorwasserstoff- oder Bromwasserstoffsäure erhalten werden, wobei diese Umsetzung vorzugsweise in alkoholischem Reaktionsmilieu erfolgt.

Für die Umsetzung geeignete quartäre Ammonium- bzw. Phosphoniumsalze sind beispielsweise solche der Formel

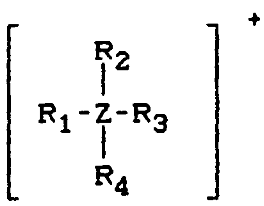

$A^{(-)}$

in welcher

| | |
|---|---|
| Z | für Stickstoff oder Phosphor steht, |
| $R_1$, $R_2$, $R_3$ und $R_4$ | die bereits obengenannte Bedeutung bzw. bevorzugte Bedeutung haben und |
| $A^{(-)}$ | für das Anion einer starken Mineralsäure mit Ausnahme der Fluorwasserstoffsäure, insbesondere für ein Chlorid- oder Bromidion steht. |

Typische Beispiele geeigneter Ausgangsmaterialien b) sind die den oben beispielhaft genannten quartären Fluoriden entsprechenden quartären Ammoniumchloride bzw. -bromide oder, jedoch weniger bevorzugt, die solchen Ammoniumsalzen entsprechenden Phosphoniumsalze.

Zur Herstellung der Katalysatoren wird beispielsweise das Alkalifluorid a) in Alkoholen wie Methanol oder Ethanol gelöst oder suspendiert und nachfolgend unter Rühren eine äquimolare Menge an Ammonium- oder Phosphoniumsalz b) zugesetzt, wobei die Mengen an a) und b) auch bis zu 50 Mol-% jeweils unterschüssig oder überschüssig sein können. Temperatur und Rührzeit sind unkritische. Normalerweise wird bei Raumtemperatur 20 bis 60 Min gerührt, das Verhältnis Komponente a) Alkalifluorid zu Alkohol beträgt üblicherweise 0,1 Mol zu 100 - 400 g Alkohol.

Eine Ausführungsform zur Herstellung der Katalysatoren besteht im nachfolgenden "Aufziehen" auf Trägermaterialien (heterogener Katalysator). Hierzu wird das beschriebene Gemisch in Alkohol, vorzugsweise von unlöslichen Bestandteilen abgetrennt, mit dem vorgesehenen Trägermaterial innig verrührt und anschließend der Alkohol, z. B. im Vakuum, abgetrennt. Geeignete Trägermaterialien sind z. B. Kieselgele, Aluminiumoxide oder Zeolithe. Kieselgele sind bevorzugt. Die Menge des Trägermaterials wird so gewählt, daß 0,05 bis 5 mMol $F^-$, vorzugsweise 0,1 bis 2 mMol $F^-$ pro g Trägermaterial resultieren.

Natürlich kann das Katalysatorsystem, von unlöslichen Bestandteilen befreit, auch als homogener Katalysator nach Abtrennen des Alkohols durch entsprechendes Verdünnen in Lösungsmitteln verwendet werden. Geeignete Lösungsmittel sind z. B. 2-Ethylhexandiol-1,3, Acetonitril oder Dimethylformamid. Die Menge des Lösungsmittels wird so gewählt daß 0,005 bis 0,5 mMol $F^-$, vorzugsweise 0,01 bis 0,1 mMol $F^-$ pro g Lösung resultieren.

Auch bei den nach diesen Methoden hergestellten Katalysatoren gilt die bereits obengemachte Aussage, derzufolge die Ammoniumfluoride gegenüber den Phosphoniumfluoriden bevorzugt sind.

Die Menge des beim erfindungsgemäßen Verfahren eingesetzten Katalysators hängt vom eingesetzten Ausgangsdiisocyanat und von der Art des Katalysators (in Lösungsmittel gelöster bzw. auf Trägermaterial aufgezogener Katalysator) ab. Die jeweils notwendige Katalysatormenge läßt sich daher am einfachsten in einem Vorversuch bestimmend. Sie liegt bei der homogenen Katalyse in der Regel bei 0,05 bis 0,3 mMol $F^-$/Mol Diisocyanat, bei der Heterogenkatalyse normalerweise bei 0,1 bis 1 mMol $F^-$/Mol Diisocyanat. Bei diesem Katalysatortyp auf Trägermaterialien können aber auch höhere Mengen verwendet werden, da durch Abfiltration der Reaktionsfortgang beliebig gestoppt werden kann.

In bestimmten Fällen kann es sich als zweckmäßig herausstellen, die katalytische Wirkung der beispielhaft genannten Katalysatoren durch eine geringe Menge an cokatalytisch wirkenden Urethangruppen zu unterstützen. Eine derartige Cokatalyse ist beispielsweise durch Zusatz einer geringen Menge eines Alkohols (beispielsweise 0,01 bis 1 Gew.-%, bezogen auf das Gewicht des eingesetzten Ausgangsdiisocyanats) erreichbar, da die zugesetzten Alkohole sofort mit dem im Überschuß vorliegenden Ausgangsdiisocyanat unter Urethanbildung abreagieren. Geeignete derartige potentielle Cokatalysatoren sind beispielsweise Methanol, Ethanol, Ethylenglykol oder 2-Ethylhexandiol-1,3. Die Alkohole können gleichzeitig mit dem Hauptkatalysator oder vorab zugesetzt werden.

Für das erfindungsgemäße Verfahren geeignete Ausgangsdiisocyanate sind beliebige organische Diisocyanate mit aliphatisch und/oder cycloaliphatisch gebundenen Isocyanatgruppen des Molekulargewichtsbereichs 140 bis 300 oder deren Gemische. Beispiele sind 1,4-Diisocyanatobutan, 1,6-Diisocyanatohexan (HDI), 1,5-Diisocyanato-2-methylpentan, 1,6-Diisocyanato-2,2,4-trimethyl-hexan, 1,12-Diisocyanatododecan, 1,3-Diisocyanatocyclobutan, 1,3-und/oder 1,4-Diisocyanatocyclohexan, 3,3'-Dimethyl-4,4'-diisocyanatodicyclohexylmethan, 4,4'-Diisocyanatodicyclohexylmethan, 1-Isocyanato-3,3,5-trimethyl-5-isocyanatomethylcyclohexan. Besonders bevorzugt wird 1,6-Diisocyanatohexan (HDI) als Ausgangsdiisocyanat verwendet.

Das erfindungsgemäße Verfahren kann sowohl lösungsmittelfrei, als auch in Anwesenheit von inerten Lösungs- und Verdünnungsmitteln durchgeführt werden. Als inerte Lösungsmittel können unpolare Verdünnungsmittel wie Toluol, Xylol oder höhere Aromaten, aber auch inerte polare Lösungsmittel wie Ester und Ketone bzw. Gemische derartiger Lösungsmittel eingesetzt werden.

Neben der Verwendung der beispielhaft genannten Katalysatoren ist die Anwesenheit von Kohlendioxid während des erfindungsgemäßen Verfahrens von erfindungswesentlicher Bedeutung. Diese Anwesenheit von Kohlendioxid wird im allgemeinen durch zumindest zeitweises Einleiten von gasförmigem Kohlendioxid in das Reaktionsgemisch während der Durchführung des erfindungsgemäßen Verfahrens sichergestellt. Vorzugsweise wird das erfindungsgemäße Verfahren unter ständigem Durchleiten eines Kohlendioxidstromes durchgeführt, so daß das Reaktionsgemisch durchgängig Kohlendioxid-gesättigt ist und ein Kohlendioxidüberschuß aus dem Reaktionsgemisch entweicht.

Das erfindungsgemäße Verfahren wird im allgemeinen im Temperaturbereich zwischen 10 bis 150°C, vorzugsweise 20 bis 100°C durchgeführt. Die optimale Reaktionstemperatur richtet sich nach der Art des gewünschten Verhältnisses Oxadiazintrion (I) zu Isocyanurat (II). Generell gilt bei gleichem Ausgangsisocyanat, gleichbleibender $CO_2$-Zugabe und gleichem Katalysator, daß sich die Verhältnisse (I) : (II) mit steigender Temperatur in Richtung Isocyanuratgruppenanteil (II) verschieben. Diese Tendenz wird durch Verringerung der $CO_2$-Zugabe naturgemäß verstärkt. Demgegenüber bleibt der Iminooxazindionanteil (III) von untergeordneter Größe. So kann jedes Verhältnis (I) : (II) innerhalb des beanspruchten Temperaturbereichs durch einen einfachen Vorversuch ermittelt werden. Im allgemeinen liegen in den erfindungsgemäßen Verfahrensprodukten, wie aus $^{13}C$-NMR-spektroskopischen Untersuchungen hervorgeht für jeden Isocyanuratring 0,01 bis 10 Oxadiazintrionringe vor.

Die erfindungsgemäße Umsetzung wird im allgemeinen bei Erreichen eines Umsetzungsgrades (Umsetzungsgrad = Prozentsatz der umgesetzten Isocyanatgruppen, bezogen auf Gesamtmenge der in Ausgangsdiisocyanat vorliegenden Isocyanatgruppen) von 10 bis 70 % abgebrochen. Der Verlauf der Reaktion kann z.B. durch fortlaufende Bestimmung des Brechungsindex' oder NCO-Wertes verfolgt werden.

Bei der lösungsmittelfreien Durchführung des erfindungsgemäßen Verfahrens, gegebenenfalls unter anschließender Entfernung von überschüssigem Ausgangsisocyanat, beispielsweise im Dünnschichtverdampfer, liegt der Umsetzungsgrad im allgemeinen zwischen 10 und 40 %. Bei der Durchführung des erfindungsgemäßen Verfahrens in Gegenwart von Lösungsmitteln ohne anschließende Entfernung von nicht umgesetztem Ausgangsisocyanat liegt der Umsetzungsgrad im allgemeinen zwischen 50 und 70 %.

Die Art der Beendigung der Umsetzungsreaktion hängt vom eingesetzten Katalysatortyp ab. Bei der homogenen Katalyse werden zur Beendigung der Umsetzungsreaktion geeignete Katalysatorgifte verwendet, wie z.B. organische Säuren bzw. Säurechloride, die die Katalysatoren in ihrer Wirkung desaktivieren, z. B. Sulfonsäuren wie beispielsweise Benzol- oder Toluolsulfonsäure oder ihre Chloride oder saure Ester der phosphorigen Säure oder der Phosphorsäure wie beispielsweise Dibutylphosphit, Dibutylphosphat oder Di-(2-ethylhexyl)-phosphat. Die beispielhaft genannten, mit den Katalysatoren unter deren Desaktivierung chemisch reagierenden Desaktivatoren werden dem Reaktionsgemisch im allgemeinen in einer dem Katalysator zumindest äquivalenten Menge zugefügt. Da die Katalysatoren jedoch während der Umsetzung teilweise desaktiviert werden, ist oftmals die Zugabe einer unteräquivalenten Menge des Desaktivators, bezogen auf die zu Beginn zugesetzte Katalysatormenge, ausreichend.

Selbstverständlich können auch Substanzen zur Beendigung der Trimerisierungsreaktion zugesetzt werden, die den Katalysator adsorptiv binden, wie beispielsweise Kieselgele oder Bleicherden, die anschließend, z. B. durch Filtration, zu entfernen sind.

Bei der Heterogenkatalyse wird, wie bereits dargelegt, die Beendigung der Reaktion durch Abtrennen des Heterogenkatalysators bewirkt.

Die erfindungsgemäßen Verfahrensprodukte können insbesondere im Falle der lösungsmittelfreien Durchführung des erfindungsgemäßen Verfahrens in an sich bekannter Weise, beispielsweise durch Dünnschichtdestillation oder Extraktion von überschüssigen, nicht-umgesetzten Ausgangsisocyanaten befreit werden, so daß Isocyanuratgruppen aufweisende Polyisocyanate mit einem Gehalt an monomeren Ausgangsisocyanaten unter 2 Gew.-%, vorzugsweise unter 0,5 Gew-%, erhältlich sind.

Die Entfernung von überschüssigen Ausgangsisocyanaten erfolgt vorzugsweise, wenn eine Anwendung der Verfahrensprodukte für Polyurethanlacke vorgesehen ist. Vor ihrer Verwendung als Isocyanat-Komponente in Zweikomponenten-Polyurethanlacken können die erfindungsgemäßen Verfahrensprodukte noch modifiziert werden, beispielsweise durch Einführung von Urethan- Harnstoff-, Biuret- oder Allophanatgruppen.

Selbstverständlich ist eine Verwendung der erfindungsgemäßen Verfahrensprodukte auch ohne Entfernung von überschüssigen Ausgangsisocyanaten möglich, beispielsweise zur Herstellung von Polyurethanschaumstoffen.

Die erfindungsgemäßen Verfahrensprodukte können selbstverständlich in an sich bekannter Weise mit geeigneten Blockierungsmitteln für Isocyanatgruppen wie z. B. Phenol, ε-Caprolactam, Malonsäurediethylester oder Acetessigsäureethylester blockiert werden.

Die erfindungsgemäßen Verfahrensprodukte bzw. ihre durch die genannte Blockierungsreaktion erhaltenen Derivate stellen wertvolle Ausgangsmaterialien zur Herstellung von Polyurethankunststoffen nach dem Isocyanat-Polyadditionsverfahren dar. Sie eignen sich insbesondere als Isocyanatkomponente in Polyurethanlacken, insbesondere in Zweikomponentenpolyurethanlacken.

Die folgenden Beispiele erläutern die Erfindung. Alle Prozentangaben betreffen, soweit nicht anderslautend ausgeführt, Gewichtsprozente.

Beispiele

A) Herstellung von Katalysatoren in Lösung

Beispiel 1

2,3 g Kaliumfluorid in 50 g Ethanol werden unter Rühren bei Raumtemperatur mit 16 g ®Aliquat 336[1)], in 60 g Ethanol gelöst, versetzt. Nach 60 min. filtriert man von unlöslichen Bestandteilen ab, fügt dem Filtrat 150 g 2-Ethylhexandiol-1,3 hinzu und trennt vom Ethanol im Vakuum ab. Die Katalysatorlösung hat die Daten:
$F^-$: 0,21 mMol/g
$Cl^-$: >0,01 mMol/g

Beispiel 2

2,9 g Kaliumfluorid in 70 g Ethanol werden unter Rühren bei Raumtemperatur mit einer Lösung aus 7,4 g Benzyltrimethylammoniumchlorid in 40 g Ethanol umgesetzt. Nach 30 min. filtriert man von unlöslichen Bestandteilen ab, fügt dem Filtrat 300 g 2-Ethylhexandiol-1,3 hinzu und trennt vom Ethanol im Vakuum ab:
$F^-$: 0,11 mMol/g
$Cl^-$: 0,02 mMol/g

Beispiel 3

2 Gew.-% Benzyltrimethylammoniumfluorid (Fa. Fluka GmbH) in 2-Ethylhexandiol-1,3:
$F^-$: 0,11 mMol/g

B) Herstellung von Katalysatoren auf Trägermaterialien

Beispiel 4

11,6 g Kaliumfluorid in 250 g Ethanol werden bei Raumtemperatur mit einer Lösung aus 81 g ®Aliquat 336 in 150 g Ethanol versetzt. Nach 60 min. filtriert man von unlöslichen Bestandteilen ab und verrührt das Filtrat mit 200 g Kieselgel 60 (Fa. Merck, 70-230 mesh, ASTM). Nach 60 min. Rühren wird das Ethanol im Vakuum abgetrennt.
Man erhält ein rieselfähiges Katalysatorsystem mit den Daten:

[1)] handelsübliches quartäres Ammoniumchlorid (Fa. Fluka GmbH) bestehend im wesentlichen aus N-Methyl-N,N,N-trialkylammoniumchlorid mit $C_8$-$C_{10}$-Alkylresten

$F^-$: 0,58 mMol/g
$Cl^-$: 0,03 mMol/g

Beispiel 5

6,2 g Kaliumfluorid in 200 g Ethanol werden bei Raumtemperatur mit einer Lösung aus 19,7 g Benzyltrimethylammoniumchlorid in 100 g Ethanol verrührt. Nach 20 min. filtriert man von unlöslichen Bestandteilen ab und verrührt das Filtrat mit 200 g Kieselgel 60 (Fa. Merck, 70 bis 230 mesh, ASTM). Nach 60 min. Rühren wird das Ethanol im Vakuum abgetrennt.

Man erhält ein rieselfähiges Katalysatorsystem mit den Daten:
$F^-$: 0,42 mMol/g
$Cl^-$: 0,04 mMol/g

C) Erfindungsgemäßes Verfahren

Beispiel 6

840 g (5 Mol) HDI werden unter Durchleiten eines Kohlendioxidstromes, der so bemessen ist, daß durchgängig ein leichter Kohlendioxidüberschuß durch einen Blasenzähler aus dem Reaktionsgefäß entweicht, auf 40°C erwärmt. Anschließend läßt man unter fortgesetztem Einheiten von Kohlendioxid 3 g Katalysator gemäß Beispiel 1 unter Rühren bei 40°C eintropfen, weitere 1 g Katalysator nach 1 Stunde und nach weiteren 2 Stunden nochmals 0,5 g Katalysator. Nach insgesamt 8 Stunden Rührzeit bei 40°C ist ein Brechungsindex von $n_D^{23}$ °C = 1.4606 erreicht. Die Lösung wird durch Zugabe von 0,2 g Phosphorsäuredibutylester abgestoppt, mit 10 g getrockneter Bleicherde 30 min. verrührt und abfiltriert. Das Filtrat wird nachfolgend durch Dünnschichtdestillation (Typ "Kurzwegverdampfer") bei 120°C/0,1 mbar bis auf einen Restgehalt von 0,1 % HDI von überschüssigem HDI abgetrennt. Man erhält ein klares, farbloses Polyisocyanat mit den Daten:
Ausbeute: 168 g (20 %)
NCO-Gehalt: 22,2 %
Viskosität 24°C: 1700 mPas
Zusammensetzung nach $^{13}C$-NMR (Mol-%):
Oxadiazintrion (I): 68 %; Isocyanurat (II): 26 %;
Iminooxazindion (III): 6 %.

Beispiel 7

Gemäß Beispiel 6 wird bei 60°C Reaktionstemperatur und verringerter Katalysatormenge (2 g, 0,5 g, 0,2 g) verfahren. Nach 6 Stunden bei 60°C ist ein Brechungsindex von $n_D^{23}$ °C = 1,4630 erreicht. Nach Aufarbeitung gemäß Beispiel a erhält man ein klares, farbloses Polyisocyanat mit den Daten:
Ausbeute: 205 g (24,5 %)
NCO-Gehalt: 22,1 %
Viskosität 24°C: 1600 mPas
Zusammensetzung nach $^{13}C$-NMR (Mol-%):
(I): 40 %; (II): 45 %; (III): 15 %.

Beispiel 8

Gemäß Beispiel 6 wird bei 80°C und einmaliger Zugabe von 2 g Katalysator verfahren. Nach 3,5 Stunden ist ein Brechungsindex von $n_D^{23}$ °C = 1,4619 erreicht. Das aufgearbeitete, klare, farblose Polyisocyanat weist folgende Daten auf:
Ausbeute: 180 g (21,5 %)
NCO-Gehalt: 23,0 %
Viskosität 24°C: 1200 mPas
Zusammensetzung nach $^{13}C$-NMR (Mol-%):
(I); 20 %; (II): 58 %; (III): 18 %.

Beispiel 9

Gemäß Beispiel 6 wird bei 100°C Reaktionstemperatur und verringerter Katalysatormenge (1,5 g 0,2 g, 0,1 g) verfahren. Nach 5 Stunden ist ein Brechungsindex $n_D^{23}$ °C = 1,4604 erreicht. Das aufgearbeitete, klare, farblose Polyisocyanat weist folgende Daten auf:
Ausbeute: 154 g (18,4 %)
NCO-Gehalt: 23,5 %
Viskosität 24°C: 1000 mPas
Zusammensetzung nach $^{13}$C-NMR (Mol-%):
(I): 14 %; (II) : 63 %; (III) : 18 %.

Beispiel 10

840 g (5 Mol) HDI werden unter Durchleiten eines Kohlendioxidstroms, der so bemessen ist, daß durchgängig ein leichter Kohlendioxidüberschuß durch einen Blasenzähler aus dem Reaktionsgefäß entweicht, auf 80°C erwärmt. Anschließend fügt man unter Rühren 2 g Katalysator gemäß Beispiel 4 bei 80°C hinzu, weitere 0,5 g Katalysator nach 2 Stunden und nach einer weiteren Stunde nochmals 0,5 g Katalysator. Nach insgesamt 5 Stunden Rührzeit bei 80°C ist ein Brechungsindex von $n_D^{23}$ °C = 1,4620 erreicht. Man filtriert vom Heterogenkatalysator ab, versetzt das Filtrat unter Rühren und ohne Heizen mit 10 g getrockneter Bleicherde, rührt 30 min nach und filtriert ab.

Das Filtrat wird nachfolgend durch Dünnschichtdestillation (Typ "Kurzwegverdampfer") bei 120°C/0,1 mbar bis auf einen Restgehalt von 0,1 % HDI von überschüssigem HDI abgetrennt. Man erhält ein klares, farbloses Polyisocyanat mit den Daten:
Ausbeute: 170 g (21 %); NCO-Gehalt: 21,6 %
Viskosität 24°C: 1600 mPa.s
Zusammensetzung nach $^{13}$C-NMR (Mol-%)
Oxadiazintrion (I): 4 %; Isocyanurat (II): 95 %

Beispiel 11

Gemäß Beispiel 10 werden bei 80°C 1 g 2-Ethylhexandiol-1,3 und 1,4 g Katalysator gemäß Beispiel 4 zugefügt, nach 3 Stunden Rührzeit bei 80°C nochmals 0,6 g Katalysator Nach insgesamt 6 Stunden Rührzeit bei 80°C ist ein Brechungsindex von $n_D^{23}$ °C = 1,4620 erreicht. Man filtriert vom Heterogenkatalysator ab, versetzt das Filtrat unter Rühren und ohne Heizen mit 10 g getrockneter Bleicherde, rührt 30 min. nach und filtriert ab.

Das Filtrat wird nachfolgend durch Dünnschichtdestillation (Typ "Kurzwegverdampfer") bei 120°C/0,1 mbar bis auf einen Restgehalt von 0,1 % HDI von überschüssigem HDI abgetrennt. Man erhält ein klares, farbloses Polyisocyanat mit den Daten:
Ausbeute: 172 g (21,9 %)
NCO-Gehalt: 23,3 %
Viskosität 24°C: 1500 mPa.s
Zusammensetzung nach $^{13}$C-NMR (Mol-%)
Oxadiazintrion (I): 3 %
Isocyanurat (II): 97 %

## Patentansprüche

**1.** Verfahren zur Herstellung von modifizierten, Isocyanuratgruppen aufweisenden Polyisocyanaten mit aliphatisch und/oder cycloaliphatisch gebundenen Isocyanatgruppen durch chemische Modifizierung eines Teils der Isocyanatgruppen von organischen Diisocyanaten mit aliphatisch und/oder cycloaliphatisch gebundenen Isocyanatgruppen in Gegenwart eines, die Trimerisierung beschleunigenden Katalysators und Abbruch der Reaktion beim jeweils erwünschten Umsetzungsgrad, dadurch gekennzeichnet, daß man

(i) als Katalysator quartäre Ammonium- oder Phosphoniumfluoride verwendet und

(ii) die Modifizierungsreaktion in Gegenwart von zumindest zeitweise während der Umsetzung in das Reaktionsgemisch eingeleitetem Kohlendioxid durchführt.

2. Verfahren gemäß Anspruch 1, dadurch gekennzeichnet, daß man nach Beendigung der Reaktion nicht umgesetztes Ausgangsdiisocyanat durch Dünnschichtdestillation entfernt.

3. Verfahren gemäß Anspruch 1 und 2, dadurch gekennzeichnet, daß man als quartäre Ammonium- oder Phosphoniumfluoride solche verwendet, wie sie durch Umsetzung von Alkalifluoriden mit quartären Ammonium- oder Phosphoniumsalzen anderer Säuren als der Fluorwasserstoffsäure erhalten werden.

4. Verfahren gemäß Anspruch 3, dadurch gekennzeichnet, daß man als Katalysatoren quartäre Ammonium- oder Phosphoniumfluoride verwendet, wie sie durch Umsetzung von Natrium- oder Kaliumfluorid mit Ammonium- oder Phosphoniumsalzen der Formel

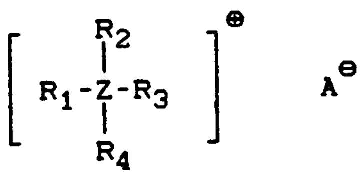

in einem alkoholischen Reaktionsmedium erhalten werden können, wobei in dieser Formel

| | |
|---|---|
| Z | für Stickstoff oder Phosphor steht, |
| $R_1$, $R_2$, $R_3$ und $R_4$ | für gleiche oder verschiedene Reste stehen und Alkylgruppen mit 1 bis 18 Kohlenstoffatomen bedeuten, wobei einer der Reste auch für einen araliphatischen Rest mit 7 bis 15 Kohlenstoffatomen stehen kann, und wobei die Summe der Kohlenstoffatome der vier Reste vorzugsweise bei 10 bis 40 liegt, und |
| $A^{\ominus}$ | für das Anion einer starken Mineralsäure mit Ausnahme der Flußsäure, insbesondere für ein Chlorid- oder Bromid steht. |

5. Verfahren gemäß Anspruch 1 bis 4, dadurch gekennzeichnet, daß man als organisches Diisocyanat 1,6-Diisocyanatohexan verwendete.

6. Verwendung der gemäß Anspruch 1 bis 5 erhältlichen modifizierten, Isocyanuratgruppen aufweisenden Polyisocyanate, gegebenenfalls in mit Blockierungsmitteln für Isocyanatgruppen blockierter Form, als Isocyanatkomponente in Polyurethanlacken.

**Claims**

1. A process for the production of modified isocyanurate polyisocyanates containing aliphatically and/or cycloaliphatically bound isocyanate groups by partial chemical modification of the isocyanate groups of organic diisocyanates containing aliphatically and/or cycloaliphatically bound isocyanate groups in the presence of a catalyst which accelerates the trimerization reaction and termination of the reaction at the particular conversion required, characterized in that
   (i) ammonium or phosphonium fluorides are used as catalyst and
   (ii) the modification reaction is carried out in the presence of carbon dioxide introduced into the reaction mixture at least periodically during the reaction.

2. A process as claimed in claim 1, characterized in that, on completion of the reaction, unreacted starting diisocyanate is removed by thin-layer distillation.

3. A process as claimed in claims 1 and 2, characterized in that the quaternary ammonium or phosphonium fluorides used are obtained by reaction of alkali metal fluorides with quaternary ammonium or phosphonium. salts of acids other than hydrofluoric acid.

4. A process as claimed in claim 3, characterized in that the catalysts used are quaternary ammonium or phosphonium fluorides of the type obtainable by reaction of sodium or potassium fluoride with ammonium or phosphonium salts corresponding to the following formula

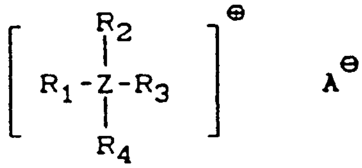

in which

| | |
|---|---|
| Z | is hydrogen or phosphorus, |
| $R_1$, $R_2$, $R_3$ and $R_4$ | may be the same or different and represent $C_{1-8}$ alkyl groups; one of the substituents may also be a $C_{7-15}$ araliphatic radical and the total of carbon atoms in the four substituents is preferably from 10 to 40, |
| $A^{\ominus}$ | is the anion of a strong mineral acid except hydrofluoric acid, more especially a chloride or bromide ion, in an alcoholic reaction medium. |

**5.** A process as claimed in claims 1 to 4, characterized in that 1,6-diisocyanatohexane is used as the organic diisocyanate.

**6.** The use of the modified isocyanurate polyisocyanates obtainable by the process claimed in claims 1 to 5, optionally blocked with blocking agents for isocyanate groups, as isocyanate component in polyurethane paints.

## Revendications

**1.** Procédé de préparation de polyisocyanates modifiés, présentant des groupes isocyanurates avec des groupes isocyanates liés à des radicaux aliphatiques et/ou cycloaliphatiques par modification chimique d'une partie des groupes isocyanates des di-isocyanates organiques avec des groupes isocyanates liés à des radicaux aliphatiques et/ou cycloaliphatiques en présence d'un catalyseur accélérant la trimérisation et avec interruption de la réaction quand le taux de transformation souhaité est atteint, caractérisé en ce que:
(i) on utilise comme catalyseur des fluorures d'ammonium ou de phosphonium quaternaires et
(ii) on réalise la réaction de modification en présence de dioxyde de carbone introduit dans le mélange réactionnel pendant la transformation au moins pendant une partie du temps.

**2.** Procédé selon la revendication 1, caractérisé en ce qu'après l'achèvement de la réaction, le diisocyanate de départ non transformé est éliminé par distillation en couches minces.

**3.** Procédé selon les revendications 1 et 2, caractérisé en ce que l'on utilise comme fluorures d'ammonium ou de phosphonium quaternaires, ceux qui sont obtenus en faisant réagir des fluorures alcalins avec des sels d'ammonium ou de phosphonium quaternaires d'acides autres que l'acide fluorhydrique.

**4.** Procédé selon la revendication 3, caractérisé en ce que l'on utilise comme catalyseurs des fluorures d'ammonium ou de phosphonium quaternaires qui peuvent être obtenus en faisant réagir du fluorure de sodium ou de potassium avec des sels d'ammonium ou de phosphonium de la formule

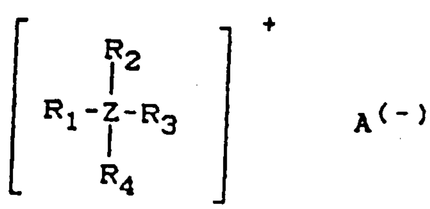

dans un milieu réactionnel alcoolique, tandis que, dans cette formule:
Z représente de l'azote ou du phosphore,

$R_1$, $R_2$, $R_3$ et $R_4$ représentent des radicaux identiques ou différents et des groupes alkyle avec de 1 à 18 atomes de carbone tandis que l'un des radicaux peut représenter également un radical araliphatique avec de 7 à 15 atomes de carbone, tandis que le total des atomes de carbone des quatre radicaux est compris, de préférence, entre 10 et 40 et

$A^{(-)}$ représente l'anion d'un acide minéral fort à l'exception de l'acide fluorhydrique et représente, en particulier, un chlorure ou bromure.

**5.** Procédé selon les revendications 1 à 4 caractérisé en ce que l'on utilise, comme diisocyanate organique, du 1,6-diisocyanatohexane.

**6.** Utilisation des polyisocyanates modifiés présentant des groupes isocyanurates obtenus conformément aux revendications 1 à 5, éventuellement sous forme bloquée pour les groupes isocyanates par des agents de blocage comme composants isocyanates dans les vernis au polyuréthane.